# EUROPEAN PATENT APPLICATION

(11) **EP 3 467 619 A2**
(43) Date of publication of application: **10.04.2019**
(21) Application number: 17794440.2
(22) Date of filing: 25.05.2017
(51) Int. Cl.: G06F 3/01, G06K 9/00, G06F 3/0484, G06F 3/03

(54) **DEVICE FOR INFLUENCING VIRTUAL OBJECTS OF AUGMENTED-REALITY**

(30) Priority: 06.06.2016 RU 2016122239
(71) Applicant: Devar Entertainment Limited, Nicosia, P.C. 1075 (CY)
(72) Inventor: Averyanov, Vitaly Vitalyevich, Tula 300005 (RU); Komissarov, Andrey Valeryevich, Tula 300024 (RU)
(74) Representative: Schmidt, Steffen J.
(86) International application number: PCT/RU2017/050042
(87) International publication number: WO 2017/213558

(57) **Abstract**

The invention refers to devices, intended for impact on virtual objects, namely to devices for impact on virtual objects augmented reality containing the which housed a video camera, display, connected to the computational module, processing device, the device has a database storage of actions virtual objects of augmented reality, correlated with different commands, correlated in advance to options of facial expressions and gestures of the user connected to the computing module that has an electronic unit adapted to recognize commands stored in the base data in the information received through a video camera of the device, wherein the output of the recognition unit connected to the entrance of the computing module of the electronic unit for activation of actions of virtual objects of augmented reality, relevant recognized commands corresponding to various facial expressions and gestures the user. According to the invention, the command recognition block further comprises a module for determining heart rate of the user. The technical result achieved is extended capabilities to influence virtual objects of augmented reality.

## Description

### TECHNICAL FIELD OF THE INVENTION

The invention relates to devices for influencing virtual objects, namely devices for influencing virtual objects of augmented reality, comprising a housing in which a video camera, display, microphone is connected to a computing unit that processes data.

The following terms are used in this paper.

**Virtual object** - a nonexistent object created by technical means from wherein sensations transmitted to a person (through hearing and vision, etc...) therefrom are simulated by technical means.

**Augmented reality** - perceived mixed reality created by using the elements "augmented" by the computer perceived reality (where virtual objects are mounted in the perceptual field).

**Device for creating and viewing objects augmented reality** - any computing device having a display and a video camera, which can transmit the display image from the camera in real time and display additional virtual image. A typical representative of such a device: smartphone, tablet computer, a computer with a headset in the form of points of augmented reality, such as Google Glass and the like.

**Smartphone** (English a smartphone - Smart phone) - a cell phone, having functionality of a Pocket PC.

**Biometric parameters of a face** - a set of specific parameters, points on the human face, which may carry by means of image analysis recognition of basic emotions expressed by mimics of a human face such as - joy, sadness, fear, surprise, anger, contempt and repulsion, as well as the signals given by face of a person (wink and stuff).

**Biometric parameters gestures** - a set of specific parameters, the points of the human body, especially the hands, wherein analyzing images of which allows recognition of signals of human gestures (stroking, parting, shaking, etc.).

### Prior Art

Currently, an increasing number of people use various electronic devices and interact with virtual objects. This happens not only in computer games, but also in the learning process, as well as, for example, in the remote trade of goods, when the buyer decides to purchase using a virtual model of goods. The most promising direction of development looks like the creation of augmented reality - that is, the combination of the display of the computer device or smart phone and glasses virtual or augmented reality, virtual objects with the real image obtained in real time time from a video camera of said device.

Besides simple observation of augmented reality objects, there is a need to interact with them, i.e. to send control signals by different means which lead to the fact that the augmented reality object is responsive to the influence.

There are known devices for influencing virtual augmented reality objects containing a housing in which a video camera and display are connected to a computing unit that processes data. This prior art is disclosed in the publication of a utility model patent of RF Nº138628 20.03.2014

This device is the closest in technical essence and achieved technical result and is chosen as a prototype of the proposed invention. Similarly to the present invention, the prototype may display virtual objects of augmented reality.

The disadvantage of this prototype is its inability to control actions or movements of the augmented reality object, depending on the commands corresponding to the facial expressions and gestures of the user.

### Description of the Invention

The technical problem addressed by the present invention is proposing a device for influencing virtual objects of augmented reality, which, at least, mitigates at least one of the above disadvantages, namely, to extend the possibility of affecting virtual objects of augmented reality by influencing virtual objects of augmented reality by facial expressions and user gestures.

To achieve this goal, the apparatus has a storage unit comprising a database of actions of virtual objects of augmented reality correlated with various commands corresponding to certain predetermined options of facial expressions and user gestures, coupled to the computing module which includes an electronic recognition unit for the various options of facial expressions and gestures of a user, received through the camera of the device, commands from the database, wherein the output of the recognition unit is connected to an input of the electronic unit, located in the computing module, which activates corresponding actions of virtual objects of augmented reality associated to recognized commands corresponding to various embodiments of facial expressions and gestures of the user.

Thanks to these advantageous characteristics, it becomes possible to manage the objects of augmented reality by mimicry and user gestures. Depending on the facial expression, gestures, the virtual object of augmented reality will perform actions corresponding to the specified command. For example, a virtual dog in augmented reality will lie down following the gesture of an outstretched hand facing downwards. A virtual person in augmented reality based on a recognized smile of the user will smile in response. A virtual kitten in augmented reality will murch when stroked by hand.

There is an embodiment of the invention in which the recognition unit of facial expressions and gestures and commands of the user has a module for recognizing the biometric parameters of the face. Thanks to this advantageous characteristic, it becomes possible to define, among the user's facial expressions, certain facial expressions that are in the database and which allow to form the corresponding commands that correspond to this user's facial expressions, said commands influencing virtual objects of augmented reality.

There is also an embodiment of the invention in which the recognition unit for facial expressions and gestures and commands of the user has a biometric gesture recognition module. With this favorable characteristic it is possible to detect user gestures among certain gestures that are in the database, and that allow them to generate corresponding commands that correspond to the gestures of the user for influencing the virtual objects of augmented reality.

There is also an embodiment of the invention, wherein the recognition unit for facial expressions and gestures and commands of the user command has a module for detecting temperature coupled with an infrared camera or thermal imager. In this case, objects of augmented reality can react to the temperature of the surrounding world, for example, at a temperature of minus twenty degrees on the street, they depict that they freeze or turn into an icicle.

Due to this advantageous characteristic, it becomes possible to determine the temperature of individual areas of the user's body, mainly faces. This in turn allows us to determine the distribution of "hot" and "cold" areas, in comparison of their localization. A quantitative estimate can also be made to determine the temperature difference (gradient) indices of the investigated area in comparison with the symmetric zone. Also, mathematical processing of the image can be performed. Orienters in the analysis of the image can serve universal features of the face: the eyebrow, the ciliary edge of the eyelids, the contour of the nose.

There is also an embodiment of the invention in which the recognition unit for facial expressions, gestures and commands of the user has a module for determining frequency of blinking of the user's eyes. Thanks to these advantageous characteristics, it becomes possible to recognize certain combinations of the blinkings of the eyes that are stored in the database and which can be interpreted as certain commands, for example, a wink with one or two eyes. Or one can track the movement of the eyeball, allowing even users with impaired motor functions to enter commands using gestures performed by movement of the eyes.

There is still a further embodiment of the invention in which the recognition unit for facial expressions, gestures and commands of the user has a module for determining a heart rate of the user.

Thanks to these advantageous characteristics, it is possible to additionally determine the heart rate and use it to improve the accuracy of recognition of the basic emotions expressed by facial expressions of the person, such as joy, sadness, fear, surprise, anger, and so on.

In addition, there is an embodiment of the invention, wherein recognition unit for facial expressions, gestures and commands of the user has a user action prediction module.

Thanks to this advantageous characteristic, it becomes possible to recognize user's facial expressions and gestures in real time, that is, even before the end of the gesture process. For example, the user just started to smile, as the module predicts the user's actions and automatically sends a signal that the user's smile is detected even before the smile itself is formed.

### Brief Description of the Drawings

Other features and advantages of the present invention clearly follow from the description given below for illustration, which is not restrictive, with references to the attached drawings, wherein:
- Figure 1 is a schematic diagram of an apparatus for influencing the virtual objects of augmented reality according to the invention,
- Figure 2 schematically shows steps of a method of influencing the virtual objects of augmented reality according to the invention.

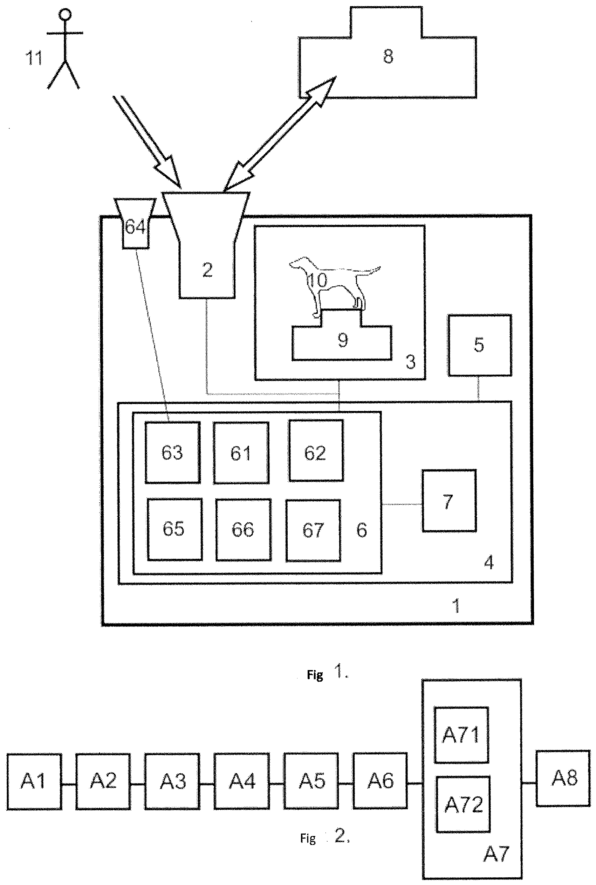

According to Figure 1 a device for influencing virtual objects of augmented reality comprises a housing 1, which accommodates a video camera 2, a display 3, connected to the computing unit 4 for processing data.

The device has a database unit for storing actions of virtual objects of augmented reality correlated with various commands, corresponding to certain variants of predetermined facial expressions and gestures of a user, said database unit connected to a computing unit 4, which comprises an electronic recognition unit 6 for correlating commands in the database with various facial expressions and gestures of the user, received from the video camera 2 of the device, wherein the output of the recognition unit 6 is connected to the input of the electronic unit 7 for activating actions of the virtual reality objects corresponding to the recognized commands corresponding to various facial expressions and gestures of the user

The user facial expression, gestures and commands recognition unit 6 may have:
- a biometric face recognition module 61,
- a biometric gestures recognition module 62
- a user temperature determination module 63 coupled with an infrared camera 64 (or imager)
- a user eye blink frequency detection unit 65,
- a user heart rate determination unit 66,
- a user actions prediction unit 67.

Figure 1 also indicates:
8 - a real object that the camcorder 2 shoots,
9 - an image of a real object on the display 3,
10 - an image of a virtual object of augmented reality on the display 3,
11 - a user.

### Detailed Description of the Invention

The device for influencing virtual objects of augmented reality works as follows. Here is the most comprehensive example of the invention, bearing in mind that this example does not limit the invention.

According to FIG. 2:
**Step A1.** Form a database of actions of virtual objects of augmented reality correlated with various commands corresponding to various embodiments, facial expressions and gestures of the user before beginning influencing virtual objects of augmented reality.
**Step A2.** Establishing in advance a correspondence between the facial expressions and gestures of the user and the variant of the actions of the augmented reality object.
**Step A3.** Locating any image in the field of view of the video camera of the device to create and view virtual objects augmented reality, said image serving as a marker for creating virtual objects augmented reality or a physical object.
**Step A4.** Creating an augmented reality object and display it on the device's display.
**Step A5.** The user shows facial expressions and gestures that are available in the database.
**Step A6.** The device takes an image of the user's face or his gesture. Accordingly, capture of a video from the camera 2 is performed.
**Step A7.** Then, by recognition unit, among the various received through the video camera of the device facial expressions and user gestures, recognizing commands from the database, said recognition is real time oriented;

Process of recognition of facial expressions may consist of several sub-steps.

**Step A71.** First, digital images are pre-processed to improve recognition quality.

**Step A72.** Then, a person's face is detected in a panoramic image and the person's image is copied to a separate frame, which is fed to a classifier's input. A neural network algorithm trained by backpropagation can be used as the classifier. The training set can comprise seven standards of the Ekman classifier, wherein mimic pictures are significantly different in expression strength.

**Step A8.** With the help of the electronic unit 7, the actions of virtual objects of augmented reality corresponding to the recognized commands corresponding to various facial expressions and gestures of the user are activated.

The apparatus can be trained, ie. new gestures can be added to the database.

To place objects of augmented reality on real objects (for example, on a table), the following operations can be performed:
1. Identifying markers of real three-dimensional space from the images obtained from the video camera of the device adapted to create and view the augmented reality. In general, a marker can be any shape or object. But in practice, we are limited by the resolution of the web-camera (phone), color rendering, lighting, and processing power of the equipment, as everything happens in real time, and therefore must be processed quickly, and therefore usually a black and white marker of simple form is selected.
2. Forming a physical base coordinate system tied to the spatial position of the markers of a real three-dimensional space.
3. Setting coordinates of the three-dimensional virtual objects of augmented reality in the base coordinate system.
4. Determining coordinates of the device adapted to create and view the augmented reality relative to the basic coordinate system by analyzing the image from the camera of the device.

The sequence of stages is exemplary and allows one to rearrange, subtract, add or perform some operations simultaneously without losing the ability to interact with virtual objects of augmented reality.

### Industrial applicability

The proposed device for influencing virtual objects of augmented reality can be implemented by a person skilled in the art and, when implemented, ensures the realization of the claimed designation, which makes it possible to conclude that the criterion "industrial applicability" for the invention is met.

In accordance with the present invention, a prototype device is manufactured. The prototype tests showed that it allows:
- determining among a variety of options of facial expressions and gestures of the user, those options that correspond to certain predefined facial expressions and gestures of the user that are pre-stored in the database,
- determining in the database of the sequence of actions of the augmented reality object corresponding to a certain gesture or facial expressions of the user,
- performing said sequence of actions corresponding to a certain gesture or facial expressions of the user, on an object of augmented reality.

**Implementation embodiment 1.** A virtual dog created as an object of augmented reality licks hands when a user is trying to pet it. See Figure. 1.
**Implementation embodiment 2.** A flower created as an object of augmented reality blooms when recognizing joy and fade when recognizing sorrow on the user's face.
**Implementation embodiment 3.** A virtual man created as an object of augmented reality waves it's hand as a greeting or farewell in response to recognizing gestures of greeting or farewell of the user.

Accordingly, this invention addresses the technical problem set - expansion of capability to interact with virtual reality objects by influencing said virtual objects of augmented reality by user's mimicry and gestures.

## Claims

1. A device for influencing virtual objects of augmented reality comprising at least:
a housing,
a camera (2),
a display (3),
a computing module (4),
a memory,
a unit (6) adapted to recognize facial expression and gestures of a user (11) the user's facial expressions and gestures recognition (11), comprising a module adapted to determine a heart rate of the user;
said device is adapted to:
capture images from the camera (2),
recognize facial expressions and gestures of the user (11),
determine heartbeat of the user (11), and
control virtual objects (10) based on recognized facial expressions and gestures, and / or a particular heartbeat of the user (11).

2. The device of claim 1, wherein the memory includes a database (5) storing actions of the virtual reality objects (10) of augmented reality correlated with commands corresponding to certain facial expressions and gestures of the user (11).

3. The device of claim 2, wherein the unit (6) is adapted to recognise commands stored in said database, wherein said commands correspond to facial expressions and gestures the user (11).

4. The device of claim 3, wherein an output of the command recognizing unit (6) is connected to an input of an electronic unit (7), located in the computing module (4), which activates corresponding actions of virtual objects of augmented reality associated to the recognized commands corresponding to various embodiments of facial expressions and gestures of the user (11).

5. The device of claim 3, wherein the recognition unit (6) also comprising at least one of:
a module (61) adapted for recognizing biometric parameters of a face of the user (11),
a module (62) adapted for recognizing biometric parameters of gestures of the user (11),
a module (63) adapted for determining temperature of the user (11) connected to an infrared camera (64) or a thermal imager,
a user eye blinking frequency detection unit (65), and
a user action prediction unit (67).
